(19) Europäisches Patentamt
European Patent Office
Office européen des brevets

(11) Veröffentlichungsnummer : **0 298 338 B1**

# (12) EUROPÄISCHE PATENTSCHRIFT

(45) Veröffentlichungstag der Patentschrift :
**24.07.91 Patentblatt 91/30**

(51) Int. Cl.$^5$ : **A01N 43/82**, B27K 3/50, C07D 285/12

(21) Anmeldenummer : **88110256.0**

(22) Anmeldetag : **28.06.88**

## (54) Mikrobizide Mittel.

(30) Priorität : **07.07.87 DE 3722320**

(43) Veröffentlichungstag der Anmeldung :
**11.01.89 Patentblatt 89/02**

(45) Bekanntmachung des Hinweises auf die Patenterteilung :
**24.07.91 Patentblatt 91/30**

(84) Benannte Vertragsstaaten :
**CH DE FR GB IT LI SE**

(56) Entgegenhaltungen :
**DE-A- 2 123 023**
**DE-A- 2 533 604**
**DE-A- 2 853 196**
**DE-C- 1 817 069**
**US-A- 3 562 284**

(73) Patentinhaber : **BAYER AG**
**W-5090 Leverkusen 1 Bayerwerk (DE)**

(72) Erfinder : **Diehr, Hans-Joachim Dr.**
**Höhe 35**
**W-5600 Wuppertal 11 (DE)**
Erfinder : **Kuck, Karl-Heinz Dr.**
**Heerstrasse 24**
**W-4018 Langenfeld (DE)**
Erfinder : **Paulus, Wilfried Dr.**
**Deswatinesstrasse 90**
**W-4150 Krefeld 1 (DE)**
Erfinder : **Schmitt, Hans-Georg Dr.**
**Am Oberend 13**
**W-4150 Krefeld 1 (DE)**

EP 0 298 338 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft die Verwendung von bestimmten in 2,5-Stellung substituierten 1,3,4-Thiadiazolen als Mikrobizide zum Schutz technischer Materialien.

Aus der DE-OS 1817069 (= US-PS 4061645) ist bekannt, daß bestimmte in 2,5-Stellung substituierte Thiadiazole wirksame Fungizide gegen im Boden, auf Samen und anderen Pflanzenteilen ansässige schädliche Pilze sind.

Aus der US-PS 3562284 ist die Wirkung dieser Verbindungen gegen humanpathogene Pilze bekannt.

Es wurde jetzt gefunden, daß diese in 2,5-Stellung substituierten 1,3,4-Thiadiazole eine überraschend hohe Wirksamkeit und ein außerordentlich breites Wirkungsspektrum gegenüber Mikroorganismen, wie Bakterien, Algen, Schleimorganismen und Pilze, entfalten, die den Abbau oder die Veränderung technischer Materialien bewirken und sich deshalb hervorragend als Mikrobizide zum Schutz technischer Materialien eignen.

Die Erfindung betrifft daher die Verwendung von in 2,5-Stellung substituierten 1,3,4-Thiadiazolen der Formel

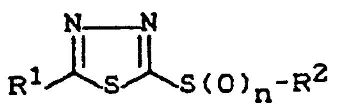

(I) ,

in welcher

$R^1$ für geradkettiges oder verzweigtes Halogenalkyl steht,
$R^2$ für geradkettiges oder verzweigtes Alkyl steht und
n für die Zahlen 1 oder 2 steht,

als Mikrobizide zum Schutz technischer Materialien.

Bevorzugt werden diejenigen Verbindungen der Formel (I) verwendet, in der n die angegebene Bedeutung hat und

$R^1$ für geradkettiges oder verzweigtes $C_1$-$C_4$-Halogenalkyl mit 1 bis 9 gleichen oder verschiedenen Halogenatomen und
$R^2$ für geradkettiges oder verzweigtes $C_1$-$C_8$-Alkyl steht.

Als $C_1$-$C_4$-Halogenalkylreste seien beispielsweise genannt:
Dichlorfluormethyl, Chlordifluormethyl, Trifluormethyl, Trichlormethyl, Chlormethyl, Dichlormethyl, 1-Chlorethyl, 2-Fluormethylpropyl und 1,3-Dichlor-2-methyl-propyl.

Besonders bevorzugte Halogenalkylreste sind:
Dichlorfluormethyl, Difluorchlormethyl, Trifluormethyl, Trichlormethyl, Chlormethyl und Dichlormethyl.

Als $C_1$-$C_8$-Alkylreste seien genannt:
Methyl, Ethyl, n-Propyl, iso-Propyl, n-Butyl, sek.-Butyl, tert.-Butyl, Pentylreste, wie der n-Pentyl-und der neo-Pentylrest, n-Hexyl, n-Heptyl und der n-Octylrest.

Besonders bevorzugte Alkylreste sind:
Methyl, Ethyl, n-Propyl und iso-Propyl.

Als Vertreter der erfindungsgemäß zu verwendenden 2,5-substituierten 1,3,4-Thiadiazole der Formel (I) seien beispielsweise genannt:

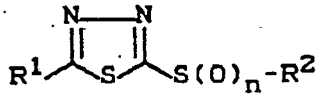

(I)

Tabelle 1

| $R^1$ | n | $R^2$ | Schmelzpunkt (°C) Brechungsindex |
|---|---|---|---|
| $CF_3$ | 2 | $CH_3$ | 86 |
| $CCl_3$ | 1 | $CH_3$ | 109 |
| $CCl_3$ | 2 | $CH_3$ | 118 |
| $CCl_2F$ | 1 | $CH_3$ | 56-57 |
| $CCl_2F$ | 2 | $CH_3$ | 57 |
| $CClF_2$ | 2 | $CH_3$ | 60 |
| $CH_2Cl$ | 2 | $CH_3$ | 57 |
| $CH_2Cl$ | 1 | $CH_3$ | $n_D^{20}$ = 1,5948 |
| $CCl_3$ | 2 | $C_3H_7$-n | 80 |
| $CHCl_2$ | 2 | $CH_3$ | 79 |
| $CHCl_2$ | 1 | $CH_3$ | 89 |
| 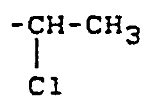 | 2 | $CH_3$ | $n_D^{20}$ = 1,5455 |
| 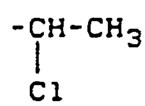 | 1 | $CH_3$ | 83 |
| $CCl_3$ | 2 | $C_8H_{17}$-n | 109 |
| $CCl_3$ | 2 | $C_2H_5$ | 68 |
| 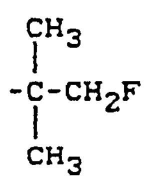 | 2 | $CH_3$ | $n_D^{20}$ = 1,5086 |
| 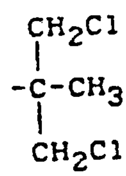 | 2 | $CH_3$ | $n_D^{20}$ = 1,522 |

Die Herstellung der erfindungsgemäß zu verwendenden 1,3,4-Thiadiazole der Formel I ist in den US-PS 4005213 und 3562284 und ein Verfahren zur Herstellung der als Ausgangsverbindungen benötigten 5-Mercapto-1,3,4-thiadiazole z.B. in der US-P 3562284 beschrieben.

Die erfindungsgemäß zu schützenden technischen Materialien sind nicht lebende Materialien, die für die

Verwendung in der Technik zubereitet worden sind. Beispielsweise können technische Materialien, die durch die erfindungsgemäß zu verwendenden Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt.

Als Mikroorganismen, die einen Abbau oder eine Veränderung der technischen Materialien bewirken können und die deshalb im Materialschutz bekämpft werden, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylocuccus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten die erfindungsgemäß zu verwendenden Wirkstoffe im allgemeinen in einer Menge von 1 bis 95 Gew.-%, bevorzugt von 10 bis 75 Gew.-% bezogen auf das Gesamtgewicht der Mittel.

Die Anwendungskonzentrationen der erfindungsgemäß zu verwendenden Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0.05 bis 1,0 Gew.-%, bezogen auf das zu schützende Material.

Die erfindungsgemäß zu verwendenden Wirkstoffe können auch in Mischung mit anderen bekannten Wirkstoffen Vorliegen. Beispielsweise seien die folgenden Wirkstoffe genannt; Benzylalkoholmono(poly)hemiformal und andere Formaldehyd abspaltende Verbindungen, Benzimidazolyl-methylcarbamate, Tetramethylthiuramisulfid, Zinksalze von Dialkyldithiocarbamaten, 2,4,5,6-Tetrachlorisophthalonitril, Thiazolylbenzimidazol, Mercaptobenzthiazol, Organo-Zinnverbindungen, Methylenbisthiocyanat, Phenolderivate, wie 2-Phenylphenol, (2,2'-Dihydroxy-5,5'-dichlor)-diphenylmethan, 3-Methyl-4-chlor-phenol und 2-Thiocyanatomethylthiobenzthiazol.

Die in den nachfolgenden Beispielen verwendeten 1,3,4-Thiadiazole waren wie folgt erhalten worden:

Stufe 1 (Herstellung der Mercapto-Verbindungen)

(a) 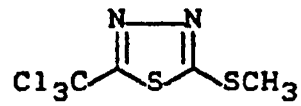

36,6g (0,3 Mol) Dithiocarbazinsäure-methylester wurden in 100 ml Diethylenglykol-dimethylether und 23,7 g (0,3 Mol) Pyridin gelöst und der Ansatz auf 0°C bis 5°C abgekühlt. Bei dieser Temperatur wurden 54,6 g (0,3 Mol) Trichloracetylchlorid zugetropft. Anschließend wurde 30 Minuten bei Raumtemperatur nachgerührt, dann bei 0°C bis 10°C 70 ml konzentrierte Schwefelsäure zugetropft und schließlich 12 Stunden bei Raumtemperatur gerührt. Das Reaktionsgemisch wurde in Eiswasser gegossen ; das wäßrige Gemisch wurde mit Toluol extrahiert. Die vereinigten Toluolextrakte wurden im Vakuum vom Toluol befreit. Der Rückstand wurde bei einer Badtemperatur von 50-60°C im Dampfstrahler andestilliert.

Ausbeute : 69,6 g (= 93,5% der Theorie) 5-Trichlormethyl-2-methylmercapto-1,3,4-thiadiazol in Form eines langsam kristallisierenden dunklen Öles erhalten.

(b) 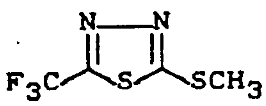

In eine Lösung von 128 g (1 Mol) Dithiocarbazinsäuremethylester (96%ig) in 260 ml Toluol und 87 g (1,1 Mol) Pyridin wurden bei −5°C bis 0°C zuerst 211 g (1,54 Mol) Phosphortrichlorid, dann 114 g (1 Mol) Trifluoressigsäure in 90 ml Toluol getropft. Nach Erwärmen auf Raumtemperatur wurde das Reaktionsgemisch 12 h bei dieser Temperatur nachgerührt und anschließend mit 100 ml konz. Schwefelsäure unter Kühlen bei Raumtemperatur versetzt. Das Gemisch wurde anschließend 2 Stunden auf 45-55°C erhitzt und nach dem Abkühlen in Eiswasser geschüttet. Das wäßrige Gemisch wurde mehrfach mit Toluol extrahiert. Die organische Phase wurde getrocknet und vom Toluol befreit. Ausbeute : 169,9 g (97%ig) ( 82,4% d.Th.) 5-Trifluormethyl-2-methylmercapto-1,3,4-thiadiazol in Form eines gelblichen Öls (Kp. 44°C/0,67 mbar).

Stufe 2a) (Herstellung der Sulfone)

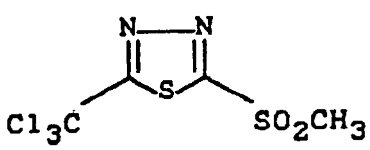

25,5 g (0,12 Mol) 2-Trichlormethyl-5-methylmercapto-1,3,4-thiadiazol und 1 g Natriumwolframat wurden in 100 ml Ameisensäure gelöst und die Lösung bei Raumtemperatur tropfenweise mit 50 ml Wasserstoffperoxid versetzt. Das Gemisch wurde auf 70°C erwärmt, 15 Minuten bei dieser Temperatur gerührt und dann auf Raumtemperatur abgekühlt. Danach wurde das Reaktionsgemisch mit Wasser versetzt und mit Dichlormethan extrahiert. Die organischen Phasen wurden über Magnesiumsulfat getrocknet und eingeengt.

Ausbeute :

23,3 g (69% der Theoerie) 2-Trichlormethyl-5-methylsulfonyl-1,3,4-thiadiazol FP. : 118°C.

Analog wurde aus dem 2-Trifluormethyl-5-methylmercapto-1,3,4-Thiadiazol das 2-Trifluormethyl-5-methylsulfonyl-1,3,4-Thiadiazol erhalten : Fp. : 86°C.

Stufe 2b (Herstellung der Sulfinylverbindung)

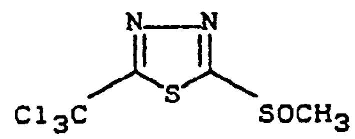

Die Lösung von 25 g (0,1 Mol) 2-Trichlormethyl-5-methylmercapto-1,3,4-thiadiazol (aus Stufe 1a) in 100 ml Dichlormethan wurde bei −60°C tropfenweise mit der Lösung von 17,3 g (0,1 Mol) m-Chlorperbenzoesäure in 150 ml Dichlormethan versetzt. Nach dem sich das Reaktionsgemisch auf Raumtemperatur erwärmt hatte, wurde es noch eine Stunde bei dieser Temperatur gerührt. Der Ansatz wurde auf Bicarbonatlösung gegossen, die organische Phase abgetrennt, gewaschen, über Natriumsulfat getrocknet und im Vakuum vom Lösungsmittel befreit. Der Rückstand wurde aus Isopropanol umkristallisiert.

Ausbeute :

18,1 g (68% der Theorie) 2-Trichlormethyl-5-methylsulfinyl-1,3,4-thiadiazol ; FP. : 109°C.

Anwendungsbeispiele

Beispiel A

Zum Nachweis der Wirksamkeit gegen Pilze werden die minimalen Hemm-Konzentrationen (MHK) erfindungsgemäß zu verwendender Wirkstoffe bestimmt :

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70% relativer Luftfeuchtigkeit wird die MHK bestimmt.

MHK ist die niedrigste Konzentration an Wirkstoff, bei der keinerlei Bewuchs durch die verwendete Mikrobenart erfolgt, sie ist in der nachstehenden Tabelle angegeben.

Tabelle A

MHK's in mg/l bei der Einwirkung erfindungsgemäß zu verwendender 1,3,4-Thiadiazole auf Pilze

| Wirkstoff / Testorganismen | 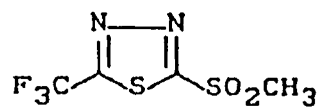 | 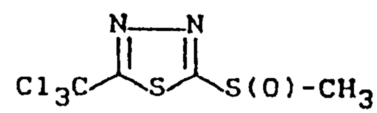 | 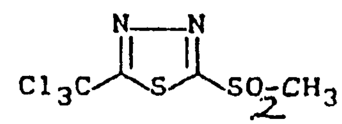 |
|---|---|---|---|
| Alternaria tenuis | 15 | 5 | 10 |
| Aspergillus niger | 20 | 50 | 20 |
| Aureobasidium pullulans | 10 | 5 | 15 |
| Chaetomium globosum | 10 | 20 | 10 |
| Cladosporium cladosporioides | 5 | 5 | 2 |
| Lentinus tigrinus | 2 | 2 | 2 |
| Penicillium glaucum | 20 | 20 | 10 |
| Sclerophoma pityophila | 5 | 5 | 10 |
| Trichoderma viride | 200 | 100 | 200 |

Beispiel B

Wirkung gegen Bakterien

Ein Agar, der als Nährmedium Bouillon enthält, wird mit erfindungsgemäß zu verwendenden Wirkstoffen in Konzentrationen von 1 bis 5000 ppm versetzt. Darauf infiziert man das Nährmedium jeweils mit den in der Tabelle B aufgeführten Testorganismen und hält das infizierte Medium 2 Wochen bei 28°C und 60 bis 70% relativer Luftfeuchtigkeit.

Die MHK ist die niedrigste Konzentration an Wirkstoff, bei der keinerlei Bewuchs durch die verwendete Mikrobenart erfolgt. Die MHK-Werte sind in Tabelle B wiedergegeben.

Tabelle B

Angabe der MHK-Werte in mg/l bei Einwirkung der unten angegebenen Wirkstoffe auf Bakterien.

| Wirkstoff / Testorganismen | 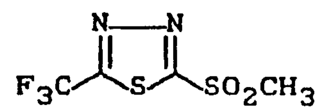 | 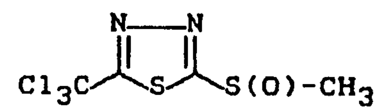 |
|---|---|---|
| Escherichia coli | 200 | 20 |
| Staphylococcus aureus | 200 | 35 |
| Pseudomonas aeruginosa | 200 | 500 |
| Pseudomonas fluorescens | 200 | 200 |
| Bacillus substilis | 500 | - |
| Bacterium punctatum | 750 | - |
| Proteus mirabilis | 100 | 20 |

Beispiel C

Wirkung gegen Schleimorganismen

Erfindungsgemäß zu verwendende Verbindungen werden in Konzentrationen von jeweils 0,1 bis 100 mg/l in Allens Nährlösung (Arch. Mikrobiol. 17, 34-53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1% Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wuden, infiziert.

Nährlösungen, die die minimale Hemmkonzentration (MHK) oder größere Wirkstoffkonzentrationen aufweisen, sind nach 3-wöchiger Kultur bei Raumtemperatur noch völlig klar, d.h. die in wirkstofffreien Nährlösungen nach 3 bis 4 Tagen bemerkbare starke Vermehrung der Mikroben und Schleimbildung unterbleibt.

Tabelle C

MHK-Wert in mg/l bei der Einwirkung der unten angegebenen Substanzen auf Schleimorganismen

| Beisp.-Nr. | MHK in mg/l |
|---|---|
| 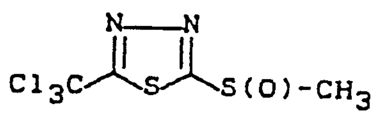 | 0,3 |
| 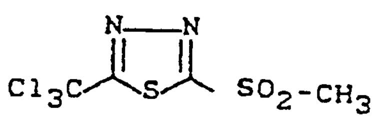 | 0,5 |
| 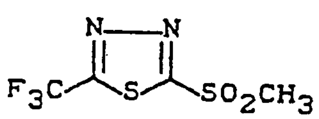 | 0,75 |

Beispiel D

Eine Mischkultur von Grün-, Blau-, Braun- und Kieselalgen (Stichococcus bacillaris Naegeli, Euglena gracilis Klebs, Chlorella pyrenoidosa Chick, Phormidium foveolarum Gomont, Oscillatoria geminata Meneghini und Phaedodactylum tricornutum Bohlin) wird unter Durchperlen von Luft in Allens Nährlösung (Arch. Mikrobiol. 17, 34-53 (1952)), die auf 4 l steriles Wasser 0,2 g Ammoniumchlorid, 4,0 g Natriumnitrat, 1,0 g Dikaliumhydrogenphosphat, 0,2 g Calciumchlorid, 2,05 g Magnesiumsulfat und 0,02 g Eisenchlorid enthält, gegeben.

Nach 2 Wochen ist die Nährlösung durch intensives Algenwachstum tief grünblau gefärbt. Das Absterben der Algen nach Zugabe erfindungsgemäß zu verwendender Wirkstoffe erkennt man an dem Entfärben der Nährlösung.

Tabelle D

Algen-abtötende Konzentrationen (mg/l) der unten angegebenen Substanzen

| Beisp.-Nr. | abtötende Konzentration in mg/l |
|---|---|
| 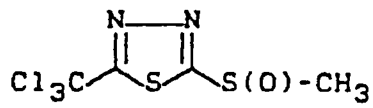 | 100 |
| 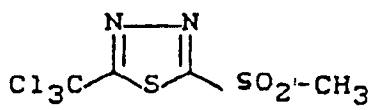 | 30 |
| 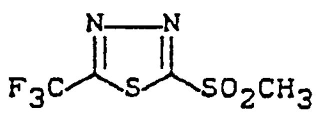 | 75 |

Beispiel E

Prüfung der Schimmelfestigkeit von Anstrichen

Die Prüfung wird in Anlehnung an den Report 219 der Defense Standards Laboratories Marigyrnong/Australien wie folgt durchgeführt :

Das zu prüfende Anstrichmittel wird beidseitig auf eine geeignete Unterlage gestrichen.

Um praxisnahe Ergebnisse zu erhalten wird ein Teil der Prüflinge vor dem Test auf Schimmelfestigkeit mit einem warmen Frischluftstrom behandelt (7 Tage ; 40°C).

Die so vorbereiteten Prüflinge werden auf einen Agar-Nährboden gelegt. Prüfling und Nährboden werden mit Pilzsporen kontaminiert. Nach 1- bis 3-wöchiger Lagerung bei 29 ± 1°C und 80-90% relativer Luftfeuchtigkeit wird abgemustert. Der Anstrich ist dauerhaft schimmelfest, wenn der Prüfling pilzfrei bleibt oder höchstens einen geringen Randbefall erkennen läßt.

Zur Kontamination werden Pilzsporen folgender neun Schimmelpilze verwendet, die als Anstrichzerstörer bekannt sind oder häufig auf Anstrichen angetroffen werden :

1. Alternaria tenuis
2. Aspergillus flavus
3. Aspergillus niger
4. Aspergillus ustus
5. Cladosporium herbarum
6. Paecilomyces variotii
7. Penicillium citrinum
8. Aureobasidium pullulans
9. Stachybotrys atra Corda

Eine Dispersionsfarbe auf Basis von PVAc, die bezogen auf Gesamtfeststoffgehalt 0,5% des Wirkstoffes (7) oder 0,5% des Wirkstoffes (8) enthält, liefert gemäß Prüfung wie oben angegeben, schimmelfesten Anstriche.

Mit dem handelsüblichen Anstrichfungizid Tetramethylthiuramdisulfid (TMTD) erzielt man in der gleichen Dispersionsfarbe diesen Effekt erst nach Einarbeitung von mindestens 3% TMTD bez. auf Gesamtfeststoffgehalt.

Anstrichmittel und Anstriche, die TMTD enthalten, verfärben, wenn sie in Kontakt mit Schwermetallspuren kommen. Anstriche und Anstrichmittel, die erfindungsgemäße Wirkstoffe enthalten, verfärben nicht.

## Patentansprüche

1. Verwendung von in 2,5-Stellung substituierten 1,3,4-Thiadiazolen der Formel

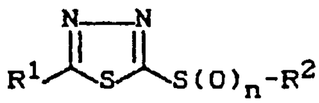

(I),

in welcher

$R^1$ für geradkettiges oder verzweigtes Halogenalkyl steht,
$R^2$ für geradkettiges oder verzweigtes Alkyl steht und
n für die Zahlen 1 oder 2 steht, als Mikrobizide zum Schutz technischer Materialien.

2. Verwendung gemäß Anspruch 1, dadurch gekennzeichnet, daß in der Formel (I)

$R^1$ für geradkettiges oder verzweigtes $C_1$-$C_4$-Halogenalkyl mit 1 bis 9 gleichen oder verschiedenen Halogenatomen,
$R^2$ für ein geradkettiges oder verzweigtes $C_1$-$C_8$-Alkyl steht.

3. Verwendung gemäß Anspruch 1 oder 2 dadurch gekennzeichnet, daß die technischen Materialien Klebstoffe, Leime, Papier, Karton, Textilien, Leder, Holz und Anstrichmittel sind.

## Claims

1. Use of 2.5-substituted 1,3,4-thiadiazoles of the formula

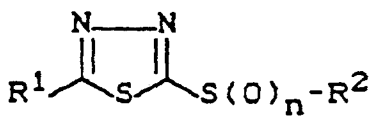

(I)

in which

$R^1$ represents straight-chain or branched halogenoalkyl,
$R^2$ represents straight-chain or branched alkyl and
n represents the numbers 1 or 2, as microbicides for protecting industrial materials.

2. Use according to Claim 1, characterised in that, in the formula (I),

$R^1$ represents straight-chain or branched $C_1$-$C_4$-halogenoalkyl having 1 to 9 identical or different halogen atoms and
$R^2$ represents a straight-chain or branched $C_1$-$C_8$-alkyl.

3. Use according to Claim 1 or 2, characterised in that the industrial materials are adhesives, glues, paper, cardboard, textiles, leather, wood and paints.

**Revendications**

1. Utilisation de 1,3,4-thiadiazoles substitués en positions 2,5 de formule

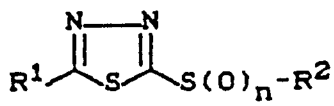

(I)

dans laquelle

$R^1$ est un groupe halogénalkyle à chaîne droite ou à chaîne ramifiée,
$R^2$ est un groupe alkyle à chaîne droite ou à chaîne ramifiée et
n représente les nombres 1 ou 2, comme microbicides pour la protection de matériaux industriels.

2. Utilisation suivant la revendication 1, caractérisée en ce que dans la formule (I)

$R^1$ est un groupe halogénalkyle en $C_1$ à $C_4$ à chaîne droite ou ramifiée ayant 1 à 9 atomes d'halogènes identiques ou différents,
$R^2$ est un groupe alkyle en $C_1$ à $C_8$ à chaîne droite ou à chaîne ramifiée.

3. Utilisation suivant la revendication 1 ou 2, caractérisée en ce que les matériaux industriels sont des adhésifs, des colles, du papier, du carton, des matières textiles, du cuir, du bois et des peintures.